# EUROPEAN PATENT SPECIFICATION

(11) **EP 4 044 194 B1**
(45) Date of publication and mention of the grant of the patent: **13.09.2023**
(21) Application number: 21156619.5
(22) Date of filing: 11.02.2021
(51) Int. Cl.: G16H 50/50, G16H 50/20, G16H 20/40

(54) **METHOD FOR ANALYSING POTENTIAL ABLATION THERAPIES**
VERFAHREN ZUR ANALYSE POTENZIELLER ABLATIONSTHERAPIEN
PROCÉDÉ D'ANALYSE DE THÉRAPIES D'ABLATION POTENTIELLES

(43) Date of publication of application: 17.08.2022
(73) Proprietor: CathVision ApS, 2200 Copenhagen (DK)
(72) Inventor: PAAMAND, Rune Tore, 3050 Humlebaek (DK); MATTHIESEN, Mads Emil, 24771 Genarp (SE)
(74) Representative: Gottschald Patentanwälte Partnerschaft mbB

(56) References cited:
- US-A1- 2017 202 471
- US-B2- 9 463 072
- TANG LISA Y W ET AL: "Predicting Catheter Ablation Outcomes with Pre-ablation Heart Rhythm Data: Less Is More", 29 September 2020 (2020-09-29), ADVANCES IN INTELLIGENT DATA ANALYSIS XIX; [LECTURE NOTES IN COMPUTER SCIENCE; LECT.NOTES COMPUTER], PAGE(S) 563 - 571, XP047590473, ISSN: 0302-9743 ISBN: 978-3-540-28540-3 * page 564, chapter 2.1; page 565, chapter 2.2; page 566, 2nd full paragraph; *
- KRUMMEN DAVID E ET AL: "Mechanisms Underlying AF: Triggers, Rotors, Other?", CURRENT TREATMENT OPTIONS IN CARDIOVASCULAR MEDICINE, CURRENT SCIENCE INC., PHILADELPHIA, PA, US, vol. 17, no. 4, 17 March 2015 (2015-03-17) , pages 1-14, XP035471252, ISSN: 1092-8464, DOI: 10.1007/S11936-015-0371-4 [retrieved on 2015-03-17]

## Description

The invention relates to a method for analysing potential ablation therapies according to claim 1, to a computer readable medium with a trained machine learning model stored on it according to the general part of claim 13, to a control system configured to perform said method according to claim 14 and to a surgery system according to claim 15.

The present method is particularly concerned with ablation therapy for atrial fibrillation. Electrically, atrial fibrillation is the chaotic activation of muscle cells of the atria. During atrial fibrillation, the atria only minimally contribute to the function of the heart. Atrial fibrillation, therefore, reduces the output of the heart but is not imminently dangerous. However, when becoming chronical, atrial fibrillation is correlated with increased morbidity and mortality. One treatment option for atrial fibrillation is ablation therapy. Ablation therapy is the destruction of the cells that allow electrical wave re-entry to reduce chaotic activation of the atrial muscle cells.

The success rate of ablation therapy depends on the location of the ablation. In many cases of atrial fibrillation, atria muscle cells around the pulmonary veins entry are ablated. While this standard therapy has shown good results for many patients, it is not always successful. For patients with complex atrial fibrillation or recurrent atrial fibrillation in particular, atrial mapping of the electrical wave fronts and re-entry points is available. However, such mapping is complex and hard to interpret. Further methods to identify and evaluate potential targets for ablation are therefore needed.

Known methods like EP 3 744 282 A2 use machine learning algorithms to generate a cardiac ablation treatment plan. However, applying machine learning to a great number of input variables requires a large training data set. It is further difficult to design a machine learning architecture that is able to generate a generic treatment plan from rather inhomogeneous input data. While considering a large number of input variables can in theory increase the precision of a machine learning algorithm, in practice, gathering the training data set from scratch by taking a large number of ablation therapies into account is unfeasible.

Other known methods (WO 2019/217430 A1) are concerned with learning activation pathways and treatment locations based on a measurement and functional models of the heart. A functional model is a good way to reduce the complexity of a machine learning algorithm and the required training data set. However, the functional understanding of atrial fibrillation is still progressing and combining functional models with machine learning algorithms is a complex task by itself.

TANG L. Y. W. et al. (2020), "Predicting Catheter Ablation Outcomes with Pre-ablation Heart Rhythm Data: Less Is More", ADVANCES IN INTELLIGENT DATA ANALYSIS XIX; pages 563 - 571, discloses a method for predicting categorical outcome information for ablation therapies based on a machine learning model.

It is therefore an object of the present invention to provide a method for analysing potential ablation therapies with a manageable complexity that is realistically trainable and usable.

The above-noted problem is solved by the method of claim 1.

The main realisation of the present invention is that pathologies of the heart that are treated with ablation are generally related to electrical misfunctions. As such, they are well described by the results of electrical measurements. It is therefore possible to develop a machine learning algorithm based on electrical data, namely ECG data. By deriving electrical biomarkers from ECG data and using a trained machine learning model to predict a change of the electrical biomarkers after applying a potential ablation therapy, the complexity of the machine learning algorithm is significantly reduced. In particular, by shifting the focus away from trying to learn a broadly defined overall success of a treatment using a multitude of data, the definition of a cost function for the training of the machine learning model becomes more easily manageable in the present invention. Being able to choose a good cost function is a key to successfully applying a machine learning algorithm.

A further advantage is that electrical measurements prior and posterior to ablation therapies are already available for a considerable number of patients. Deriving a predicted change of the electrical biomarkers also allows presenting the predicted change to a physician. This allows the physician to design an appropriate overall treatment for the patient based on the predicted change, paired with the physician's knowledge and experience. This can further enhance the quality of the overall treatment and the acceptance of the proposed method by physicians and patients.

In detail, a method for analyzing potential ablation therapies is proposed, in particular for patients with atrial fibrillation, via a control system, wherein the method comprises an analysis step in which the control system applies a trained machine learning model to input data thereby generating output data, wherein the input data comprise electrical biomarkers derived from ECG data from a patient of at least one potential ablation therapy, the potential ablation therapy including at least one potential ablation event with a potential ablation location, wherein the output data comprise a predicted change of the electrical biomarkers after applying the potential ablation therapy to the patient, wherein the predicted change is derived from the input data via the trained machine learning model.

According to claim 2 the input data may be mainly constituted by ECG-derived data or ECG-derived data and ECG-data, further enhancing the above-mentioned advantages. Claim 2 also concerns the possibility of analysing the output data to identify potential ablation targets for the patient. A predicted change in electrical biomarkers can be a good indicator for identifying potential ablation targets and planning an ablation therapy.

A training step for the machine learning model is proposed in claim 3. The training data set may be derived from a training database which can be retrospectively labelled. This database may be based on an open or commercial database thereby greatly increasing the usability of the proposed method.

In an embodiment according to claim 4 the electrical biomarkers may be derived by the control system from the ECG data of the training database.

Presently, the biomarker response to single ablation events and the whole ablation therapy is of particular interest. In the embodiment according to claim 5, a first model for deriving a change of the electrical biomarkers after applying one of the potential ablation events and/or a second model to derive a change of the electrical biomarkers may be trained in the training step. In some cases, single ablation events may have a well-defined biomarker response while in other cases the biomarker response of the ablation therapy is composed by more than one ablation event. For the latter, the single ablation events may have a synergetic effect which can be seen by considering the whole ablation therapy.

Claim 6 concerns the possibility that the first and the second model may be applied separately or jointly and in particular may be applied to different electrical biomarkers.

In an embodiment according to claim 7, the training data set may comprise focal source ablation events. Focal source ablations as a result of mapping are more specific, provide a wider range of ablation locations and enable identifying potential focal sources by the proposed method.

Claim 8 concerns a derival step for deriving electrical biomarkers from the ECG data automatically. The electrical biomarkers may be derived from the ECG data by non-machine learning algorithms. Here, known algorithms may be used to derive a number of electrical biomarkers from ECG data. In particular, those named in claim 9 are applied in the proposed method. However, it is equally preferred to use a machine learning model to derive one or more of the electrical biomarkers. This machine learning model could be part of the machine learning model for deriving the predicted change of the electrical biomarkers, thereby enabling the predictions based on ECG data directly.

In the embodiment according to claim 10, in particular depending on the available training data set, the possibilities of using mainly the last ablation event of the ablation therapies of the training database or using ablation events throughout the ablation therapies is considered.

The embodiment of claim 11 relates to determining a classification of the potential ablation therapy by using the output data, which can also be based on a machine learning algorithm. Further, the analysis of electrical biomarkers based on non-machine learning algorithms can also be utilized in the classification.

According to claim 12, the method may provide the output of a classification of potential ablation therapies, in particular from online ECG data measurements. The output can be used to identify or verify potential ablation locations even during surgery.

Another teaching according to claim 13, which is of equal importance, is directed to a computer readable medium with a trained machine learning model stored on it. All explanations given with respect to the proposed method and in particular with respect to the training step are fully applicable.

Another teaching according to claim 14, which is also of equal importance, is directed to a control system configured to perform the proposed method. All explanations given with respect to the proposed method are fully applicable.

Another teaching according to claim 15, which is also of equal importance, is directed to a surgery system connected to or forming part of the proposed control system. All explanations given with respect to the proposed method are fully applicable.

In the following, an embodiment of the invention is explained with respect to the drawings. The drawings show
- Fig. 1: the proposed method schematically,
- Fig. 2: the training of the machine learning model and
- Fig. 3: the application of the trained machine learning model.

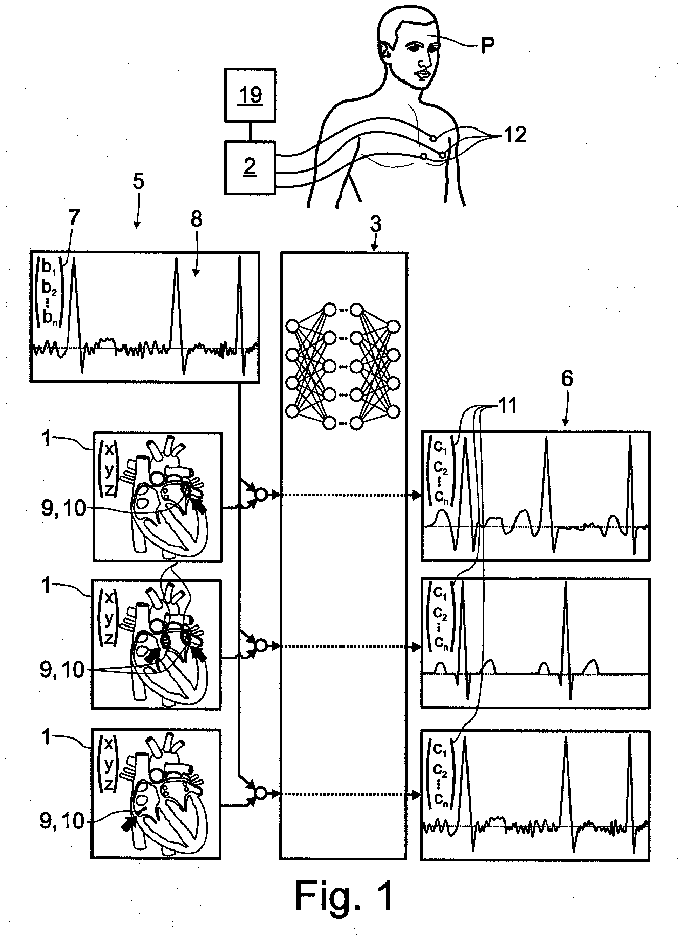

The proposed method is used for analysing potential ablation therapies 1. In the preferred case shown in the Figures, the proposed method is used for patients P with atrial fibrillation. The potential ablation therapies 1 are analysed via a control system 2. In Fig. 1, the control system 2 is shown schematically as a dedicated control system 2 comprising hardware connected to the patient P. The control system 2 may also be a general-purpose computer, a cloud-based computer system or the like and configured to perform the proposed method.

The method comprises an analysis step 3 in which the control system 2 applies a trained machine learning model 4 to input data 5 thereby generating output data 6.

Presently the term "trained machine learning model" describes at least the minimum amount of data necessary to apply the result of training of a machine learning algorithm. The trained machine learning model 4 may be present in a compressed representation and does not have to be usable by any general-purpose computer. The trained machine learning model 4 may, for example, comprise weights of an otherwise predefined artificial neural network. The necessary operations to apply the trained machine learning model 4 may be stored in the control system 2 and/or be part of the trained machine learning model 4. In a preferred embodiment, however, the trained machine learning model 4 comprises program code and is applicable to the input data 5 by a general-purpose operating system.

The input data 5 comprise electrical biomarkers 7 derived from ECG data 8 from a patient P of at least one potential ablation therapy 1. The potential ablation therapy 1 includes at least one potential ablation event 9 with a potential ablation location 10. The input data 5 are schematically shown on the left side in Fig. 1. The electrical biomarkers 7 will be explained further below in more detail. The potential ablation therapy 1 may comprise only one potential ablation event 9 or more than one potential ablation event 9. The potential ablation therapy 1, the potential ablation event 9 and the potential ablation location 10 may be stored in any suitable data structure. This data structure is preferably standardised. In Fig. 1, the values x, y, z represent the respective potential ablation therapy 1, e.g. the coordinates of a potential ablation location 10 in the potential ablation therapy 1, and are shown as an example for illustrative purposes. Fig. 1 depicts three potential ablation therapies 1, one of them comprising two potential ablation events 9. The term "potential" means that this ablation therapy 1 does not necessarily have to be applied to a patient P. Presently, the proposed method rather supports making a prediction if applying this potential ablation therapy 1 to a patient P is promising.

An ablation event 9 is formed by one or more regions in an area targeting a common focal source or by one or more regions that otherwise share a common area, for example, the entrance of a pulmonary vein. An ablation event may for example be one application of a cryocatheter.

The output data 6 comprise a predicted change of the electrical biomarkers 7 after applying the potential ablation therapy 1 to the patient P. The predicted change is derived from the input data 5 via the trained machine learning model 4. In Fig. 1, the change is shown in a set of changed electrical biomarkers 11. Further, a change in the ECG data 8 is illustrated for explanatory purposes.

The ECG data 8 are preferably surface ECG data or intracardiac ECG data and may be measured by electrodes 12 connected to the control system 2.

The electrical biomarkers 7, or some of the electrical biomarkers 7, may be time dependent and/or time independent. By choosing electrical biomarkers 7 as the focus of the machine learning mechanism and looking at the predicted change of the electrical biomarkers 7, a number of machine learning architectures become particularly suitable for the proposed method. The following list of preferred architectures is to be understood as not being comprehensive.

The trained machine learning model 4 is derived by training in a generally known manner. The trained machine learning model 4 is part of a machine learning mechanism comprising training and application of the trained machine learning model 4.

The underlying architecture may be a fixed-input architecture. Preferably the fixed-input architecture is a convolutional neural network, a feed forward network, a deep residual network or a classification architecture such as a support vector machine or a Bayes classifier. The fixed input architecture may be coupled to an autoencoder.

To generate the fixed input, the ECG data 8 may be split into time windows of a fixed length comprising one or more QRS-complexes. Additionally or alternatively, the electrical biomarker 7 may be time independent. Convolutional networks may be used for 2D input data 5. However, 1D implementations may also be applied, being well-suited for time series. Depending on the input data 5, a feed forward network is preferable, which may form a particularly simple approach. In particular, if the input data 5 comprise long time series, solutions for the vanishing gradient problem like deep residual networks can be used. In general, the ECG data 8 prior to an ablation therapy 1 may have a time dependence with early and late events being equally important. The named classification architectures may in particular be advantageous for a larger number of correlated electrical biomarkers 7.

Also preferred are recurrent architectures, in particular recurrent neural networks, more preferably, architectures based on long short-term memory or gated recurrent units, which can for example be used depending on the length of the ECG data 8 and/or the electrical biomarkers 7. Another preferred architecture is an attention architecture, in particular a transformer architecture and/or a non-recurrent architecture with an attention mechanism. In case it is not known in which part of ECG data 8 the relevant information for predicting the success of a potential ablation therapy 1 may be located, it can be advantageous to use low frequencies of ECG data 8 and derive long sequences of electrical biomarkers 7. In particular, if relevant information is sparsely distributed, attention architectures are well-suited.

The cost function for training may be based on the known response to the ablation therapy 1, as will be further described. The response can be compensated by time distance to the ablation therapy 1 and/or ablation event 9. It is preferred to predict a long time change of the electrical biomarkers 7 and/or a direct change of the electrical biomarkers 7 shortly after the ablation therapy 1.

Here and preferably, the input data 5 are mainly constituted by ECG-derived data, in particular by the electrical biomarkers 7. Alternatively, the input data 5 may be mainly constituted by ECG-derived data and ECG data 8. The term "mainly" here and in the following means that the named data form the core of the respective data or the algorithm using the respective data but other data may also be present. This takes into account that it is possible to include additional data into a machine learning algorithm without greatly influencing the result. The definition of the term "mainly" is therefore to be understood as a functional definition.

The targeted use of non-electrical data 8 may be advantageous for the proposed method. The proposed method may comprise analysing output data 6 to identify potential ablation targets for the patient P.

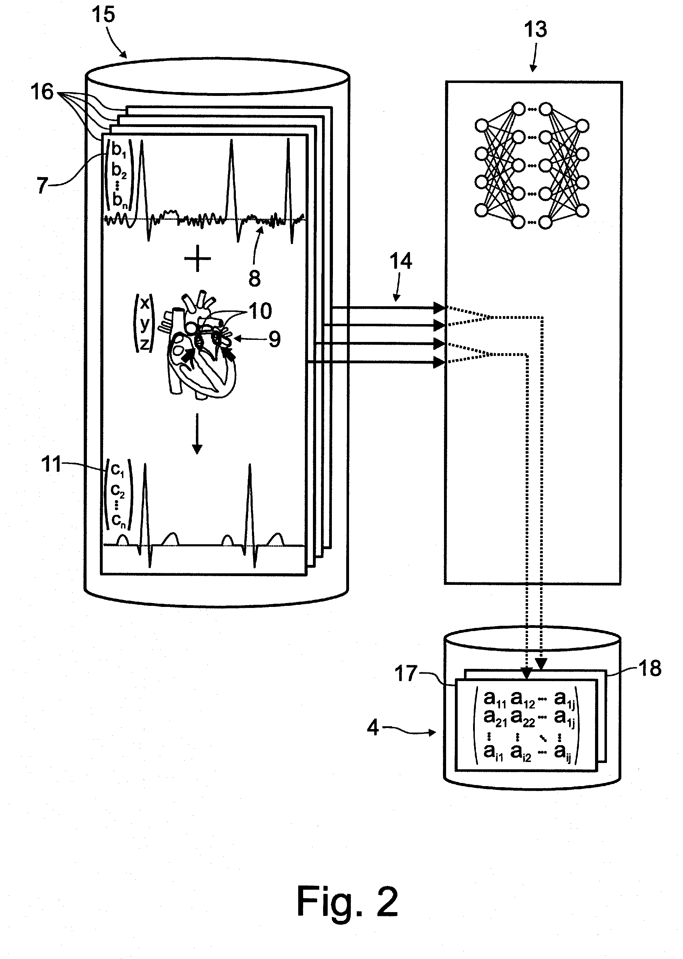

With reference to Fig. 2, the training of the machine learning model 4 will be described. Here and preferably, the trained machine learning model 4 has been or is trained in a training step 13 on a training data set 14 by the control system 2. The training data set 14 may be derived from a training database 15 that comprises ablation therapy data 16 including ablation events 9 and ECG data 8 determined prior and posterior to the ablation therapies 1 and/or one or more of the ablation events 9.

Predicting a change of electrical biomarkers 7 has the advantage that databases with a variety of ablation therapies 1 can be used for training. Therefore, preferably the training step 13 comprises retrospectively labelling the training database 15 thereby generating at least part of the training data set 14.

The labelling may in principle be performed at least partially manually. It is however preferred that the control system 2 at least partially, in particular mainly or completely, derives the electrical biomarkers 7 from the ECG data 8 of the training database 15 prior and posterior to the ablation therapies 1 and/or one or more of the ablation events 9. Automatically deriving the electrical biomarkers 7 from a database already containing ECG data 8 changes after an ablation therapy 1 enables an efficient approach for unsupervised learning.

In a preferred embodiment, the potential ablation therapy 1 includes at least two potential ablation events 9 with different ablation locations 10. In the training step 13, the control system 2 then trains a first model 17 to derive a predicted change of the electrical biomarkers 7 after applying one of the potential ablation events 9 and/or a second model 18 to derive a predicted change of the electrical biomarkers 7 of the applying potential ablation therapy. In some cases, the result of a complete ablation therapy 1 may vary from the sum of the results of single ablation events 9. In other cases, a single ablation event 9 may be the source of success of the ablation therapy 1. Looking at individual ablation events 9 and the ablation therapy 1 may therefore lead to an improved analysis.

It is noted that the training does not have to be completely automatic. The training step 13 and therefore the trained machine learning model 4 may comprise a step of automatically training and a step of manual fine-tuning. Generally, the trained machine learning model 4 may also be part of a model comprising the trained machine learning model 4 and further manually derived models.

The proposed method allows for an effective definition of a cost-function for training the trained machine learning model 4. This is advantageous as the cost function may be used with many different architectures. In one preferred embodiment, the architecture behind the machine learning itself is derived and/or changed during the training step 13. The training step 13 may include a neural architecture search and/or use guided or automated hyperparameter tuning.

For faster training of the first and the second model 17, 18, a general model may be trained and the first and the second model 17, 18 may be trained by transfer learning from the general model.

Here and preferably, in the analysis step 3 the control system 2 applies the first and the second model 17, 18 to the input data 5 to derive the predicted change of the electrical biomarkers 7. Preferably, in the analysis step 3 the control system 2 applies the first and the second model 17, 18 to a different subset of the electrical biomarkers 7 to derive a predicted subchange of the electrical biomarkers 7 after one or more of the potential ablation events 9 and after the potential ablation therapy 1. The control system 2 can then derive the predicted change from the predicted subchanges of the electrical biomarkers 7.

Regarding the training data set 14, the ablation therapies 1 in the training data set 14 may mainly comprise at least one focal source ablation event. Preferably, the training data set 14 may comprise ablation therapies 1 including mainly focal source ablation events. A focal source ablation event is an ablation event 9 for which a mapping or other determination of a focal source has been done previously to ablation therapy 1, to identify focal sources and where at least one of the focal sources is ablated. Nevertheless, an advantage of the proposed method is that a mapping is not always or even mostly not necessary. It is also not necessary to use the actual information on the mapping in the training data set 14, even if the training data set 14 comprises focal source ablation events derived from mapping.

The method may comprise a derival step in which the control system 2 applies an algorithm to the ECG data 8 to derive at least part of the electrical biomarkers 7 from the ECG data 8 automatically. Preferably, in the derival step the control system 2 applies one or more non-machine learning algorithms to the ECG data 8 to derive one or more of the electrical biomarkers 7. Additionally or alternatively, in the derival step, the control system 2 may apply one or more secondary machine learning models to the ECG data 8 to derive one or more of the electrical biomarkers 7.

Here and preferably, the same algorithm or the same architecture is used to derive the electrical biomarkers 7 for training and in the derival step.

The secondary machine learning models may be based on any suitable architecture, preferably on convolutional networks or an autoencoder. It is also possible to include the secondary machine learning model or models into the machine learning model 4 and apply and/or train these models together. For example, feature extraction layers are known.

The electrical biomarkers 7 may comprise at least one of: a cardiac cycle-length, an ECG morphology classification, a signal amplitude, a cardiac rhythm classification, a peak timing value, in particular R-peak timing value, a peak variability, in particular R-peak variability or a quantitative change between beats, in particular consecutive beats, for example a mean square error value. Additionally or alternatively, the input data 5 may comprise patient data, preferably at least one of age, sex, risk factors, and/or biological biomarkers, preferably biomarkers representative for blood pressure data and/or blood-measured biomarkers.

The cardiac cycle-length can be derived from an R-R-distance-measurement and/or a frequency analysis. The morphology can be identified by a score, a value like T-T-voltage increase, a comparison with QRS templates, or the like. Relevant is the standardised learning mechanism. Amplitude and power can be represented by mean values, peak values or the like. The rhythm classification may include a regularity score and/or a standard deviation of cycle-length.

Non-electrical biomarkers may be included into the machine learning mechanism to interpret the result of the prediction; they may be mixed with the electrical biomarkers 7 or included only in an output layer for example. Non-electrical biomarkers may also be used in the derival step.

Here and preferably, the trained machine learning model 4, in particular the first model 17, is trained by using mainly the last ablation event 9 of the ablation therapies 1 of the training database 15 as the training data set 14 or by using ablation events 9 throughout the ablation therapies 1. Here, as mentioned above with respect to the definition of the term "mainly", it would be possible to include a number of ablation events 9 that were not the last ablation events 9 in the training without creating notable adverse effects for the training data set 14.

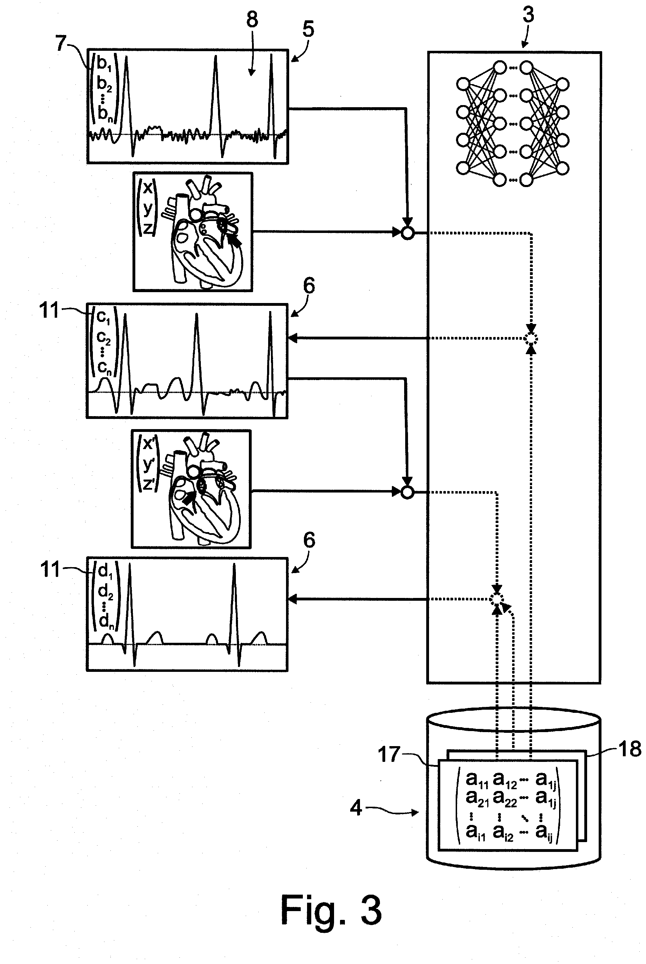

Fig. 3 shows a possible application of the first and the second model 17, 18. At first, the first model 17 is used to predict the change of the biomarkers 7 for the ablation event 9 and afterwards the first and the second model 17, 18 are used to predict the change of the biomarkers 7 after a second ablation event 9 concluding the ablation therapy 1.

The proposed method may comprise an ablation therapy classification step in which the control system 2 determines classification of one or more potential ablation events 9 and/or the potential ablation therapy 1, in particular by determining from the output data 6 a success score relating to the probability of successful treatment of the patient P by applying the potential ablation therapy 1. Preferably, the method comprises an ablation therapy determination step in which the control system 2 determines a classification for at least two potential ablation events (9) and/or at least two potential ablation therapies 1 based on the output data 6 and/or in which the control system 2 determines an optimized ablation therapy 1 based on the output data 6.

The success of a potential ablation event 9 may be classified different from the success of the potential ablation therapy 1. In particular, the control system 2 or a physician may design the potential ablation therapy by adding potential ablation events 9 targeting different electrical biomarkers. The proposed method may comprise a step of classifying potential ablation events 9 into predefined classes of potential ablation events 9. The predefined classes can then be defined as classes having one of a group of predefined results on one or more of the electrical biomarkers 7. The possibility of judging the result of a single potential ablation event 9, possibly even without the context of the complete potential ablation therapy 1, is one of the advantages of the proposed method.

The control system 2 may receive ECG data 8, preferably from an online measurement of a patient P, in particular during surgery, and output the classification of potential ablation therapies 1. Preferably the potential ablation therapies 1 are at least partly defined by measuring ECG data 8 at or near potential ablation locations 10 during surgery. In this way, it becomes possible to identify and/or verify an ablation therapy 1 during surgery. In particular, it becomes possible to include or exclude at least one potential ablation event 9 in the ablation therapy 1.

The control system 2 may receive ECG data 8 from a surgery system 19.

According to another teaching, a computer readable medium with a trained machine model 4 stored on it is proposed. Reference is made to all explanations given above. The trained machine learning model 4 on the computer readable medium has been derived in the training step 13.

According to another teaching, a control system 2 configured to perform the proposed method is proposed. Reference is made to all explanations given above.

According to another teaching, a surgery system 19 connected to or forming part of a control system 2 is proposed. The control system 2 is configured to perform the proposed method. Reference is made to all explanations given above. The surgery system 19 is adapted to be used during surgery, adapted to receive online ECG data 8 during surgery and adapted to display a result of the therapy classification step and/or the ablation therapy determination step. The surgery system may be connected to electrodes 12 for measuring the online ECG data 8. It may include hardware for processing ECG data 8, a display and/or an input unit.

## Claims

1. Method for analyzing potential ablation therapies (1), in particular for patients (P) with atrial fibrillation, via a control system (2),
wherein the method comprises an analysis step (3) in which the control system (2) applies a trained machine learning model (4) to input data (5) thereby generating output data (6),
wherein the input data (5) comprise electrical biomarkers (7) derived from ECG data (8) from a patient (P) of at least one potential ablation therapy (1), the potential ablation therapy (1) including at least one potential ablation event (9) with a potential ablation location (10),
wherein the output data (6) comprise a predicted change of the electrical biomarkers (7) after applying the potential ablation therapy (1) to the patient (P), wherein the predicted change is derived from the input data (5) via the trained machine learning model (4).

2. Method according to claim 1, **characterized in that** the input data (5) is mainly constituted by ECG-derived data, in particular by the electrical biomarkers (7), or, that the input data (5) are mainly constituted by ECG-derived data and ECG data (8), and/or, that the method comprises analyzing the output data (6) to identify potential ablation targets for the patient (P).

3. Method according to claim 1 or 2, **characterized in that** the trained machine learning model (4) has been or is trained in a training step (13) on a training data set (14) by the control system (2), preferably, that the training data set (14) is derived from a training database (15) that comprises ablation therapy data (16) including ablation events (9) and ECG data (8) determined prior and posterior to the ablation therapies (1) and/or one or more of the ablation events (9), more preferably, that the training step (13) comprises retrospectively labeling the training database (15) thereby generating at least part of the training data set (14).

4. Method according to claim 3, **characterized in that** the control system (2) at least partially, in particular mainly or completely, derives the electrical biomarkers (7) from the ECG data (8) of the training database (14) prior and posterior to the ablation therapies (1) and/or one or more of the ablation events (9).

5. Method according to claim 3 or 4, **characterized in that** the potential ablation therapy (1) includes at least two potential ablation events (9) with different ablation locations (10), that in the training step (13) the control system (2) trains a first model (17) to derive a predicted change of the electrical biomarkers (7) after applying one of the potential ablation events (9) and/or a second model (18) to derive a predicted change of the electrical biomarkers (7) after applying the potential ablation therapy (1).

6. Method according to claim 5 **characterized in that** in the analysis step (3) the control system (2) applies the first and/or second model (17, 18) to the input data (5) to derive the predicted change of the electrical biomarkers (7), preferably, that in the analysis step (3) the control system (2) applies the first and the second model (17, 18) to a different subset of the electrical biomarkers (7) to derive a predicted subchange of the electrical biomarkers (7) after one or more of the potential ablation events (9) and after the potential ablation therapy (1), and that the control system (2) derives the predicted change from the predicted subchanges of the electrical biomarkers (7).

7. Method according to one of claims 3 to 6, **characterized in that** the ablation therapies (1) in the training data set (14) mainly comprise at least one focal source ablation event, preferably that the training data set (14) comprises ablation therapies (1) including mainly focal source ablation events.

8. Method according to one of the preceding claims, **characterized in that** the method comprises a derival step in which the control system (2) applies an algorithm to the ECG data (8) to derive at least part of the electrical biomarkers (7) from the ECG data (8) automatically, preferably, that in the derival step the control system (2) applies one or more non-machine learning algorithms to the ECG data (8) to derive one or more of the electrical biomarkers (7), and/or, that in the derival step the control system (2) applies one or more trained secondary machine learning models to the ECG data (8) to derive one or more of the electrical biomarkers (7).

9. Method according to one of the preceding claims, **characterized in that** the electrical biomarkers (7) comprise at least one of: a cardiac cycle-length, an ECG morphology classification, a signal amplitude, a signal power, a cardiac rhythm classification, a peak timing value, in particular R-peak timing value, a peak variability, in particular R-peak variability or a quantitative change between beats, in particular consecutive beats, for example a mean square error value; and/or, that the input data (5) further comprises patient data, preferably at least one of: age, sex, risk factors, and/or biological biomarkers, preferably biomarkers representative for blood pressure data and/or blood-measured biomarkers.

10. Method according to one of the preceding claims, **characterized in that** the trained machine learning model (4), in particular the first model (17), is trained by using mainly the last ablation event (9) of the ablation therapies (1) of the training database (15) as the training data set (14) or by using ablation events (9) throughout the ablation therapies (1).

11. Method according to one of the preceding claims, **characterized in that** the method comprises an ablation therapy classification step in which the control system (2) determines a classification of one or more potential ablation events (9) and/or the potential ablation therapy (1), in particular by determining from the output data (6) a success score relating to the probability of successful treatment of the patient (P) by applying the potential ablation therapy (1), preferably, that the method comprises an ablation therapy determination step in which the control system (2) determines a classification for at least two potential ablation events (9) and/or at least two potential ablation therapies (1) from the output data (6), and/or, in which the control system (2) determines an optimized ablation therapy (1) from the output data (6).

12. Method according to claim 11, **characterized in that** the control system (2) receives ECG data (8), preferably from an online measurement of a patient (P), in particular during surgery, and outputs the classification of potential ablation therapies (1), preferably, that the potential ablation therapies (1) are at least partly defined by measuring ECG data (8) at or near potential ablation locations (10) during surgery.

13. Computer readable medium with a trained machine learning model (4) stored on it,
**characterized in**
**that** the trained machine learning model (4) has been derived in the training step (13) according to claim 3 and possibly one of claims 4 to 12.

14. Control system configured to perform the method according any of claims 1 to 12.

15. Surgery system connected to or forming part of a control system (2), wherein the control system (2) is configured to perform the method according to claim 11 and possibly claim 12, wherein the surgery system (19) is adapted to be used during surgery, adapted to receive online ECG data (8) during surgery and adapted to display a result of the therapy classification step and/or the ablation therapy determination step.

## Patentansprüche

1. Verfahren zum Analysieren möglicher Ablationstherapien (1), insbesondere für Patienten (P) mit Vorhofflimmern, über ein Steuerungssystem (2),
wobei das Verfahren einen Analyseschritt (3) umfasst, in dem das Steuerungssystem (2) ein trainiertes Maschinenlernmodell (4) auf Eingabedaten (5) anwendet und dadurch Ausgabedaten (6) erzeugt,
wobei die Eingabedaten (5) elektrische Biomarker (7) umfassen, die von EKG-Daten (8) eines Patienten (P) mit mindestens einer potenziellen Ablationstherapie (1) abgeleitet sind, wobei die potenzielle Ablationstherapie (1) mindestens ein potenzielles Ablationsereignis (9) mit einer potenziellen Ablationsposition (10) beinhaltet,
wobei die Ausgabedaten (6) eine vorhergesagte Veränderung der elektrischen Biomarker (7) nach dem Anwenden der potenziellen Ablationstherapie (1) auf den Patienten (P) umfassen, wobei die vorhergesagte Veränderung aus den Eingabedaten (5) über das trainierte Maschinenlernmodell (4) abgeleitet wird.

2. Verfahren nach Anspruch 1, **dadurch gekennzeichnet, dass** die Eingabedaten (5) hauptsächlich aus EKG-abgeleiteten Daten, insbesondere aus den elektrischen Biomarkern (7), besteht, oder dass die Eingabedaten (5) hauptsächlich aus EKG-abgeleiteten Daten und EKG-Daten (8) bestehen, und/oder dass das Verfahren die Analyse der Ausgabedaten {6) umfasst, um potenzielle Ablationsziele für den Patienten (P) zu identifizieren.

3. Verfahren nach Anspruch 1 oder 2, **dadurch gekennzeichnet, dass** das trainierte Maschinenlernmodell (4) in einem Trainingsschritt (13) an einem Trainingsdatensatz (14) durch das Steuerungssystem (2) trainiert wurde oder wird, bevorzugt, dass der Trainingsdatensatz (14) aus einer Trainingsdatenbank (15) abgeleitet wird, die Ablationstherapiedaten (16) umfasst, die Ablationsereignisse (9) und EKG-Daten (8) beinhaltet, die vor und nach den Ablationstherapien (1) und/oder einem oder mehreren der Ablationsereignisse (9) bestimmt wurden, bevorzugt, dass der Trainingsschritt (13) das retrospektive Markieren der Trainingsdatenbank (15) umfasst, wodurch zumindest ein Teil des Trainingsdatensatzes (14) erzeugt wird.

4. Verfahren nach Anspruch 3, **dadurch gekennzeichnet, dass** das Steuerungssystem (2) die elektrischen Biomarker (7) zumindest teilweise, insbesondere überwiegend oder vollständig, aus den EKG-Daten (8) der Trainingsdatenbank (14) ableitet und
im Anschluss an die Ablationstherapien (1) und/oder eines oder mehrere der Ablationsereignisse (9).

5. Verfahren nach Anspruch 3 oder 4, **dadurch gekennzeichnet, dass** die potenzielle Ablationstherapie (1) mindestens zwei potenzielle Ablationsereignisse (9) mit unterschiedlichen Ablationspositionen (10) beinhaltet, dass das Steuerungssystem (2) in dem Trainingsschritt (13) ein erstes Modell (17) zur Ableitung einer vorhergesagten Veränderung der elektrischen Biomarker (7) nach dem Anwenden eines der potenziellen Ablationsereignisse (9) und/oder ein zweites Modell (18) zur Ableitung einer vorhergesagten Veränderung der elektrischen Biomarker (7) nach dem Anwenden der potenziellen Ablationstherapie (1) trainiert.

6. Verfahren nach Anspruch 5, **dadurch gekennzeichnet, dass** in dem Analyseschritt (3) das Steuerungssystem (2) das erste und/oder zweite Modell (17, 18) auf die Eingabedaten (5) anwendet, um die vorhergesagte Veränderung der elektrischen Biomarker (7) abzuleiten, bevorzugt, dass in dem Analyseschritt (3) das Steuerungssystem (2) das erste und das zweite Modell (17, 18) auf eine unterschiedliche Teilmenge der elektrischen Biomarker (7) anwendet, um eine vorhergesagte Teiländerung der elektrischen Biomarker (7) nach einem oder mehreren der potenziellen Ablationsereignisse (9) und nach der potenziellen Ablationstherapie (1) abzuleiten, und dass das Steuerungssystem (2) die vorhergesagte Änderung aus den vorhergesagten Teiländerungen der elektrischen Biomarker (7) ableitet.

7. Verfahren nach einem der Ansprüche 3 bis 6, **dadurch gekennzeichnet, dass** die Ablationstherapien (1) im Trainingsdatensatz (14) hauptsächlich mindestens ein fokales Quellenablationsereignis umfassen, bevorzugt, dass der Trainingsdatensatz (14) Ablationstherapien (1) umfasst, die hauptsächlich fokale Quellenablationsereignisse beinhalten.

8. Verfahren nach einem der vorhergehenden Ansprüche, **dadurch gekennzeichnet, dass** das Verfahren einen Ableitungsschritt umfasst, in dem das Steuerungssystem (2) einen Algorithmus auf die EKG-Daten (8) anwendet, um zumindest einen Teil der elektrischen Biomarker (7) aus den EKG-Daten (8) automatisch abzuleiten, bevorzugt, dass in dem Ableitungsschritt das Steuerungssystem (2) einen oder mehrere Nicht-Maschinenlernalgorithmen auf die EKG-Daten (8) anwendet, um einen oder mehrere der elektrischen Biomarker (7) abzuleiten, und/oder dass in dem Ableitungsschritt das Steuerungssystem (2) ein oder mehrere trainierte sekundäre Maschinenlernmodelle auf die EKG-Daten (8) anwendet, um einen oder mehrere der elektrischen Biomarker (7) abzuleiten.

9. Verfahren nach einem der vorhergehenden Ansprüche, **dadurch gekennzeichnet, dass** die elektrischen Biomarker (7) umfassen: eine Herzzykluslänge, und/oder eine EKG-Morphologie-Klassifikation, und/oder eine Signalamplitude, und/oder eine Signalleistung, und/oder eine Herzrhythmus-Klassifikation, und/oder einen Peak-Timing-Wert, insbesondere R-Peak-Timing-Wert, und/oder eine Peak-Variabilität, insbesondere R-Peak-Variabilität, und/oder eine quantitative Veränderung zwischen Schlägen, insbesondere aufeinanderfolgenden, Schlägen, beispielsweise einen mittleren quadratischen Fehlerwert; und/oder dass die Eingabedaten (5) ferner Patientendaten, bevorzugt umfassen: Alter, und/oder Geschlecht, und/oder Risikofaktoren, und/oder biologische Biomarker, bevorzugt für repräsentative Biomarker für Blutdruckdaten und/oder blutgemessene Biomarker.

10. Verfahren nach einem der vorhergehenden Ansprüche, **dadurch gekennzeichnet, dass** das trainierte Maschinenlernmodell (4), insbesondere das erste Modell (17), unter Verwendung hauptsächlich des letzten Ablationsereignisses (9) der Ablationstherapien (1) der Trainingsdatenbank (15) als Trainingsdatensatz (14) oder unter Verwendung von Ablationsereignissen (9) über die gesamten Ablationstherapien (1) trainiert wird.

11. Verfahren nach einem der vorhergehenden Ansprüche, **dadurch gekennzeichnet, dass** das Verfahren einen Ablationstherapie-Klassifizierungsschritt umfasst, bei dem das Steuerungssystem (2) eine Klassifizierung eines oder mehrerer potenzieller Ablationsereignisse (9) und/oder der potenziellen Ablationstherapie (1) bestimmt, insbesondere durch Bestimmen eines Erfolgswertes aus den Ausgabedaten (6), der sich auf die Wahrscheinlichkeit einer erfolgreichen Behandlung des Patienten (P) durch Anwenden der potenziellen Ablationstherapie (1) bezieht, bevorzugt, dass das Verfahren einen Ablationstherapie-Bestimmungsschritt umfasst, bei dem das Steuerungssystem (2) aus den Ausgabedaten (6) eine Klassifizierung für mindestens zwei potenzielle Ablationsereignisse (9) und/oder mindestens zwei potenzielle Ablationstherapien (1) bestimmt, und/oder, bei dem das Steuerungssystem (2) aus den Ausgabedaten (6) eine optimierte Ablationstherapie (1) bestimmt.

12. Verfahren nach Anspruch 11, **dadurch gekennzeichnet, dass** das Steuerungssystem (2) EKG-Daten (8), bevorzugt aus einer Online-Messung eines Patienten (P), insbesondere während der Operation, empfängt und die Klassifizierung potenzieller Ablationstherapien (1) ausgibt, bevorzugt, dass die potenziellen Ablationstherapien (1} zumindest teilweise durch Messen von EKG-Daten (8) an oder in der Nähe von potenziellen Ablationspositionen (10) während der Operation definiert werden.

13. Computerlesbares Medium, auf dem ein trainiertes Maschinenlernmodell (4) gespeichert ist,
**dadurch gekennzeichnet, dass**
das trainierte Maschinenlernmodell (4) im Trainingsschritt (13) nach Anspruch 3 und gegebenenfalls einem der Ansprüche 4 bis 12 abgeleitet worden ist.

14. Steuerungssystem, das dazu ausgelegt ist, das Verfahren nach einem der Ansprüche 1 bis 12 durchzuführen.

15. Operationssystem, das mit einem Steuerungssystem (2) verbunden ist oder einen Teil davon bildet, wobei das Steuerungssystem (2) dazu ausgelegt, das Verfahren nach Anspruch 11 und gegebenenfalls Anspruch 12 durchzuführen, wobei das Operationssystem (19) angepasst ist, um während der Operation verwendet zu werden, angepasst ist, um Online-EKG-Daten (8) während der Operation zu empfangen, und angepasst ist, um ein Ergebnis des Therapieklassifizierungsschritts und/oder des Ablationstherapie-Bestimmungsschritts anzuzeigen.

## Revendications

1. Procédé d'analyse de thérapies d'ablation potentielles (1), en particulier pour des patients (P) atteints de fibrillation auriculaire, par l'intermédiaire d'un système de commande (2),
le procédé comprenant une étape d'analyse (3) au cours de laquelle le système de commande (2) applique un modèle d'apprentissage automatique entraîné (4) aux données d'entrée (5), générant ainsi des données de sortie (6),
les données d'entrée (5) comprenant des biomarqueurs électriques (7) dérivés de données ECG (8) d'un patient (P) d'au moins une thérapie d'ablation potentielle (1), la thérapie d'ablation potentielle (1) comprenant au moins un événement d'ablation potentiel (9) avec un emplacement d'ablation potentiel (10),
les données de sortie (6) comprenant un changement prédit des biomarqueurs électriques (7) après l'application de la thérapie d'ablation potentielle (1) au patient (P), le changement prédit étant dérivé des données d'entrée (5) par l'intermédiaire du modèle d'apprentissage automatique entraîné (4).

2. Procédé selon la revendication 1, **caractérisé en ce que** les données d'entrée (5) sont principalement constituées par des données dérivées de l'ECG, en particulier par les biomarqueurs électriques (7), ou, **en ce que** les données d'entrée (5) sont principalement constituées par des données dérivées de l'ECG et des données ECG (8), et/ou, **en ce que** le procédé comprend l'analyse des données de sortie (6) pour identifier des cibles d'ablation potentielles pour le patient (P).

3. Procédé selon la revendication 1 ou 2, **caractérisé en ce que** le modèle d'apprentissage automatique entraîné (4) a été ou est entraîné dans une étape d'entraînement (13) sur un ensemble de données d'entraînement (14) par le système de commande (2), de préférence, **en ce que** l'ensemble de données d'entraînement (14) est dérivé d'une base de données d'entraînement (15) qui comprend des données de thérapie d'ablation (16) comprenant des événements d'ablation (9) et des données ECG (8) déterminées avant et après les thérapies d'ablation (1) et/ou un ou plusieurs des événements d'ablation (9), plus préférablement, **en ce que** l'étape d'entraînement (13) comprend l'étiquetage rétrospectif de la base de données d'entraînement (15), générant ainsi au moins une partie de l'ensemble de données d'entraînement (14).

4. Procédé selon la revendication 3, **caractérisé en ce que** le système de commande (2) dérive au moins partiellement, en particulier principalement ou complètement, les biomarqueurs électriques (7) des données ECG (8) de la base de données d'entraînement (14) avant et après les thérapies d'ablation (1) et/ou un ou plusieurs des événements d'ablation (9).

5. Procédé selon la revendication 3 ou 4, **caractérisé en ce que** la thérapie d'ablation potentielle (1) comprend au moins deux événements d'ablation potentiels (9) avec différents emplacements d'ablation (10), **en ce que** dans l'étape d'entraînement (13) le système de commande (2) entraîne un premier modèle (17) pour dériver un changement prédit des biomarqueurs électriques (7) après l'application de l'un des événements d'ablation potentiels (9) et/ou un deuxième modèle (18) pour dériver un changement prédit des biomarqueurs électriques (7) après l'application de la thérapie d'ablation potentielle (1).

6. Procédé selon la revendication 5, **caractérisé en ce que** dans l'étape d'analyse (3) le système de commande (2) applique le premier et/ou le second modèle (17, 18) aux données d'entrée (5) pour dériver le changement prédit des biomarqueurs électriques (7), de préférence, **en ce que** dans l'étape d'analyse (3) le système de commande (2) applique le premier et le second modèle (17, 18) à un sous-ensemble différent des biomarqueurs électriques (7) pour dériver un sous-changement prédit des biomarqueurs électriques (7) après un ou plusieurs des événements d'ablation potentiels (9) et après la thérapie d'ablation potentielle (1), et **en ce que** le système de commande (2) dérive le changement prédit à partir des sous-changements prédits des biomarqueurs électriques (7).

7. Procédé selon l'une des revendications 3 à 6, **caractérisé en ce que** les thérapies d'ablation (1) dans l'ensemble de données d'entraînement (14) comprennent principalement au moins un événement d'ablation de source focale, de préférence **en ce que** l'ensemble de données d'entraînement (14) comprend des thérapies d'ablation (1) comprenant principalement des événements d'ablation de source focale.

8. Procédé selon l'une des revendications précédentes, **caractérisé en ce que** le procédé comprend une étape de dérivation dans laquelle le système de commande (2) applique un algorithme aux données ECG (8) pour dériver automatiquement au moins une partie des biomarqueurs électriques (7) à partir des données ECG (8), de préférence, **en ce que**, dans l'étape de dérivation, le système de commande (2) applique un ou plusieurs algorithmes d'apprentissage non-automatique aux données ECG (8) pour dériver un ou plusieurs des biomarqueurs électriques (7), et/ou **en ce que**, dans l'étape de dérivation, le système de commande (2) applique un ou plusieurs modèles d'apprentissage automatique secondaires entraînés aux données ECG (8) pour dériver un ou plusieurs des biomarqueurs électriques (7).

9. Procédé selon l'une des revendications précédentes, **caractérisé en ce que** les biomarqueurs électriques (7) comprennent au moins l'un des éléments suivants : une longueur de cycle cardiaque, une classification morphologique de l'ECG, une amplitude de signal, une puissance de signal, une classification du rythme cardiaque, une valeur de synchronisation du pic, en particulier la valeur de synchronisation du pic R, une variabilité du pic, en particulier la variabilité du pic R ou un changement quantitatif entre les battements, en particulier les battements consécutifs, par exemple une valeur d'erreur quadratique moyenne ; et/ou, **en ce que** les données d'entrée (5) comprennent en outre des données relatives au patient, de préférence au moins l'une des données suivantes : l'âge, le sexe, les facteurs de risque et/ou les biomarqueurs biologiques, de préférence les biomarqueurs représentatifs des données relatives à la pression artérielle et/ou les biomarqueurs mesurés dans le sang.

10. Procédé selon l'une des revendications précédentes, **caractérisé en ce que** le modèle d'apprentissage automatique entraîné (4), en particulier le premier modèle (17), est entraîné en utilisant principalement le dernier événement d'ablation (9) des thérapies d'ablation (1) de la base de données d'entraînement (15) comme ensemble de données d'entraînement (14) ou en utilisant des événements d'ablation (9) tout au long des thérapies d'ablation (1).

11. Procédé selon l'une des revendications précédentes, **caractérisé en ce que** le procédé comprend une étape de classification de thérapie d'ablation dans laquelle le système de commande (2) détermine une classification d'un ou de plusieurs événements d'ablation potentiels (9) et/ou de la thérapie d'ablation potentielle (1), notamment en déterminant à partir des données de sortie (6) un score de réussite relatif à la probabilité de réussite du traitement du patient (P) en appliquant la thérapie d'ablation potentielle (1), de préférence, **en ce que** le procédé comprend une étape de détermination de thérapie d'ablation dans laquelle le système de commande (2) détermine une classification pour au moins deux événements d'ablation potentiels (9) et/ou au moins deux thérapies d'ablation potentielles (1) à partir des données de sortie (6), et/ou, dans laquelle le système de commande (2) détermine une thérapie d'ablation optimisée (1) à partir des données de sortie (6).

12. Procédé selon la revendication 11, **caractérisé en ce que** le système de commande (2) reçoit des données ECG (8), de préférence à partir d'une mesure en ligne d'un patient (P), en particulier pendant la chirurgie, et émet la classification des thérapies d'ablation potentielles (1), de préférence, **en ce que** les thérapies d'ablation potentielles (1) sont au moins partiellement définies par la mesure des données ECG (8) aux emplacements d'ablation potentiels (10) ou à proximité de ceux-ci pendant la chirurgie.

13. Support lisible par ordinateur sur lequel est stocké un modèle d'apprentissage automatique entraîné (4), **caractérisé en ce que** le modèle d'apprentissage automatique entraîné (4) a été dérivé lors de l'étape d'entraînement (13) selon la revendication 3 et éventuellement selon l'une des revendications 4 à 12.

14. Système de commande configuré pour réaliser le procédé selon l'une quelconque des revendications 1 à 12.

15. Système de chirurgie connecté à ou faisant partie d'un système de commande (2), le système de commande (2) étant configuré pour réaliser le procédé selon la revendication 11 et éventuellement selon la revendication 12, le système de chirurgie (19) étant adapté pour être utilisé pendant la chirurgie, adapté pour recevoir des données ECG en ligne (8) pendant la chirurgie et adapté pour afficher un résultat de l'étape de classification de la thérapie et/ou de l'étape de détermination de la thérapie d'ablation.
